# EUROPEAN PATENT SPECIFICATION

(11) **EP 2 544 667 B1**
(45) Date of publication and mention of the grant of the patent: **14.11.2018**
(21) Application number: 11754004.7
(22) Date of filing: 09.03.2011
(51) Int. Cl.: A61K 9/28, A61K 9/48, A61K 9/50

(54) **ALCOHOL RESISTANT ENTERIC PHARMACEUTICAL COMPOSITIONS**
ALKOHOLRESISTENTE ENTERALE PHARMAZEUTISCHE ZUSAMMENSETZUNGEN
COMPOSITIONS PHARMACEUTIQUES ENTÉRIQUES RÉSISTANTES AUX ALCOOLS

(30) Priority: 22.07.2010 US 366825 P; 11.06.2010 US 353950 P; 09.04.2010 US 322567 P; 09.03.2010 US 312081 P; 15.04.2010 US 324656 P
(43) Date of publication of application: 16.01.2013
(73) Proprietor: Alkermes Pharma Ireland Limited, Dublin 4 (IE)
(72) Inventor: LIVERSIDGE, Gary, West Chester PA 19380 (US); MANSER, David, Longford County (IE); SHAH, Hardik, Dublin (IE); RUDDY, Stephen B., Schwenksville PA 19473 (US); REKHI, Gurvinder, Singh, Suwanee GA 30024 (US)
(74) Representative: Bailey, Sam Rogerson
(86) International application number: PCT/US2011/027736
(87) International publication number: WO 2011/112709

(56) References cited:
- EP-A2- 0 693 282
- WO-A1-2007/131357
- WO-A1-2009/076764
- WO-A2-2011/039768
- DE-U1-202006 014 131
- GB-A- 2 431 875
- US-A1- 2007 190 142
- US-A1- 2007 212 414
- US-A1- 2007 264 346
- US-A1- 2008 031 901
- US-A1- 2008 038 345
- US-A1- 2008 038 345
- US-A1- 2009 155 357
- US-A1- 2009 155 357

## Description

### BACKGROUND OF THE INVENTION

Unintended, rapid drug release in a short period of time of the entire amount or a significant portion of the drug contained in a dosage form is referred to as "dose dumping". Dose-dumping poses a significant risk to patients because of safety issues and/or diminished efficacy, particularly in controlled release dosage form where the active drug may be present in relatively high amounts. In these controlled release dosage forms, the rate of drug released from the dosage form is controlled by the release-rate-controlling mechanism. Typical release-rate-controlling mechanisms include swellable polymers, gel matrixes and polymeric coatings, to name a few. A compromise or failure of the release-rate-controlling mechanism is a likely cause of dose dumping. The likelihood of dose-dumping for certain controlled release products when administered with food has been recognized for more than twenty years. See Hendeles L, Wubbena P, Weinberger M. Food-induced dose dumping of once-a-day theophylline. Lancet. 22: 1471 (1984).

In addition to food, the presence of alcohol can compromise release-rate-controlling mechanisms of controlled release dosage forms. Certain controlled release dosage form employing release-rate-controlling mechanisms are more susceptible to dose dumping in the presence of alcohol than other release-rate-controlling mechanisms.

In 2005, the United States Food and Drug Administration (FDA) required the withdrawal of several drugs from the market or required a change in the warning labels because of the effects of ethanol on the controlled release formulations of the drug. For example, the FDA asked Purdue Pharma of Stamford, CT to withdraw Palladone® (hydromorphone hydrochloride) extended release capsules from the market because a pharmacokinetic study showed that when Palladone® was taken with alcohol, its extended release formulation was compromised and resulted in dose dumping (cf. FDA Press Release of Jul. 13, 2005). The FDA concluded that the overall risk versus benefit profile of the Palladone® drug product was unfavorable due to its alcohol induced dose dumping susceptibility. The FDA decision was based, in part, on an a pharmacokinetic study in healthy subjects (utilizing a naltrexone block), which demonstrated that co-ingestion of Palladone® with 240 mL (8 ounces) of 40% (80 proof) alcohol resulted in an average peak hydromorphone concentration approximately six times greater than when taken with water. Furthermore, one subject in this study experienced a 16-fold increase when the drug was ingested with 40% alcohol compared with water. This study also showed that 8 ounces of 4% alcohol (equivalent to 2/3 of a typical serving of beer) could in some subjects result in almost twice the peak plasma hydromorphone concentration than when the drug was ingested with water. FDA Alert for Healthcare Professionals (July 2005): Hydromorphone Hydrochloride Extended-Release Capsules (marketed as Palladone®). http://www.fda.gov/cder/drug/InfoSheets/HCP/hydromorphoneHCP.pdf.

An *in vivo* alcohol dose dumping resistance test is not the preferred approach due to potential harm the test could pose to a human subject. The preferred approach, according to the FDA, is an *in vitro* dissolution test in the presence of 40% ethanol. At the Pharmaceutical Sciences Advisory Committee Meeting of Oct. 26, 2005, OPS (Office of Pharmaceutical Science) personnel from CDER (Center for Drug Evaluation and Research) presented data showing that in an alcohol susceptible controlled release dosage form, a higher concentration of ethanol (e.g., 40%) is likely to trigger faster drug release than a lower concentration of ethanol (e.g., 20% or 4%). This may or may not be the case depending on the specifics of the controlled release formulation. (See Presentations at the Pharmaceutical Sciences Advisory Committee Meeting Oct. 26, 2005). Accordingly, the Division of Bioequivalence - 2, Office of Generic Drugs CDER/FDA on 13 May 2009 at the AAPS workshop, Physical Pharmacy and Biopharmaceutics issued proposed dissolution testing for alcohol-induced dose-dumping of generic MR oral drug products. The proposed dissolution study is designed to compare dissolution performance of the generic (test) product and the corresponding reference listed drug. Conditions for dissolution include 0.1N HCL media with differing amounts of ethanol (v/v) added to give the following percentages of ethanol in the media: 0.0%, 5.0%, 20%, and 40%. Protocols similar to these prescribed dissolution studies were adopted to ascertain the robustness of the alcohol resistant pharmaceutical composition of the present invention.

At least one attempt has been made to make a controlled release formulation resistant to ethanol-induced dose dumping. U.S. Published Patent Application No. 2007/0212414 assigned to Penwest Pharmaceuticals Co., of Patterson NY, claims a method of preventing dose-dumping of a drug in the presence of ethanol by providing a patient likely to consume ethanol while being treated with the drug an effective amount of the drug in the form of an ethanol-resistant sustained release formulation. The drug and a sustained release delivery system include at least one heteropolysaccharide gum, at least one homopolysaccharide gum, and at least one pharmaceutical diluent. This ethanol-resistant sustained release formulation is claimed to essentially retain its sustained release dissolution profile in the presence of ethanol.

Different approaches have been taken to the problem of ethanol-induced dose dumping including formulations having alcohol-resistant coatings. For example, US 2009/0155357 is directed to alcohol resistant oral dosage forms which are intended to avoid dose dumping if taken with alcohol by using various coatings. US 2008/0038345 is directed to modified release compositions comprising a bupropion salt, in particular the provision of a once daily formulation of a pharmaceutically acceptable salt of bupropion with enhanced stability to simplify the dosing regimen and improve patient compliance. US 2008/0031901 relates to monoeximic solid dosage forms for administering pharmaceutical active agents, particularly hydrocodone and acetaminophen, and aims to improve upon previous controlled release coatings by reducing the complexity thereof. US 2007/0190142 is directed to a dosage form and method for the delivery of drugs, particularly those capable of abuse by using melt-processed formulations, WO 2009/076764 describes a misuse preventative, controlled release formulation comprising a core with a superabsorbent material, a controlled release coat surrounding the core and a plurality of controlled release microparticles having a pharmaceutically active agent dispersed within the core, the coat, or both the core and the coat. When crushed and exposed to an aqueous medium, the superabsorbent material of the core swells to encapsulate the microparticles which remain substantially intact thereby retarding the release of the active from the formulation. EP 0 693 282 is directed to enteric duloxetine pellets which comprise hydroxypropylmethylcellulose acetate succinate. DE 202006014131) is directed to methods of reducing alcohol induced dose dumping for opioid sustained release oral dosage forms by employing dosage forms having an enteric coating which comprises a polymer component. WO 2007/131357 relates to an abuse-resistant pharmaceutical paste composition comprising an active, and materials selected from controlled release agents, or oily, waxy or fatty substances. GB 2 431 875 relates to methods for the sustained release administration of opioids that exhibit improved properties with respect to co-administration with alcohol, in cluding formulations using alcohol resistant coatings. US 2007/0264346 relates to an oral dosage form comprising reservoir type microparticles for modified release of an active principle, characterised in that it is resistant to immediate dumping of the active in alcohol through use, in some instances, of resistant coatings. WO 2011/039768 is directed to a pharmaceutical composition for reducing alcohol induced dose dumping. The composition comprises a core having an active, a separating layer and a functional layer comprising at least one pharmaceutically acceptable polymer and which may further comprise a rate controlling polymer. There is a need in the art for enteric coated pharmaceutical formulations that resist ethanol-induced dose dumping.

### SUMMARY OF THE INVENTION

The invention is related to an alcohol-resistant pharmaceutical composition according to claim 1 comprising: (i) an active agent; (ii) an enteric system; and (iii) an alcohol protectant in an amount sufficient to prevent release of the active agent in the presence of alcohol, the alcohol protectant being organic based cellulose acetate phthalate; wherein the alcohol protectant is present in the pharmaceutical composition in an amount that provides a percentage weight gain ranging from 10% to 500%, and wherein the percentage of active agent released is less than or about 35% in 40% ethanolic HCl in 2 hrs.

Also described is a composition having an alcohol protectant that prevents release of the active agent from the composition when placed in an alcohol environment in an amount that is less than the amount of active agent released by the same composition without the alcohol protectant in the same alcohol environment.

Also described is such a composition for use in a method of treating a disease.

Also described is an alcohol resistant pharmaceutical composition having an active agent and an alcohol protectant, which alcohol protected formulation has a similar in vitro dissolution profile in 40% ethanolic acid (0.1N HCl) for 2 hours (USP I or III) followed by phosphate buffer pH 6.8 (USP I or II) for 4 hours when compared to a commercially equivalent product.

Also described is an alcohol protected formulation that bioequivalent to a commercially equivalent product.

### BRIEF DESCRIPTION OF THE DRAWINGS

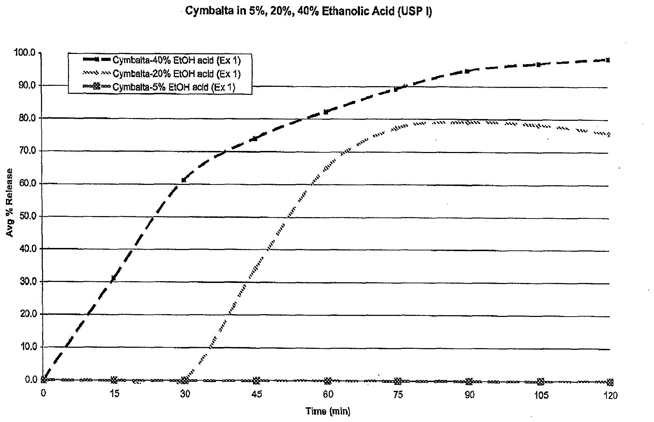

Fig 1 is a plot of the average released amount of drug, duloxetine hydrochloride(% released) over time (min) in 5%, 20%, and 40% ethanolic acid of uncoated, commercially available Cymbalta® beads (Example 1).

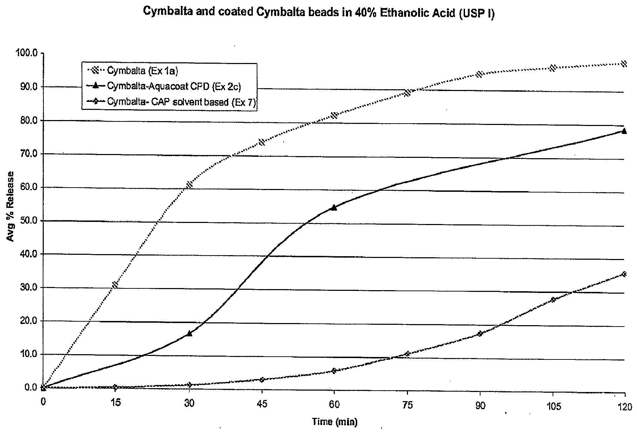

Fig. 2 is a plot of the average released amount of drug, duloxetine (% released) over time (min) in 40% ethanolic acid of (1) uncoated, commercially available Cymbalta® beads (Example 1); (2) Cymbalta® beads coated with aqueous-based CAP (AQUACOAT®-CPD by FMC Biopolymer of Philadelphia, PA) (Example 2C); and (3) Cymbalta® beads coated with organic-based CAP dispersion (Example 7).

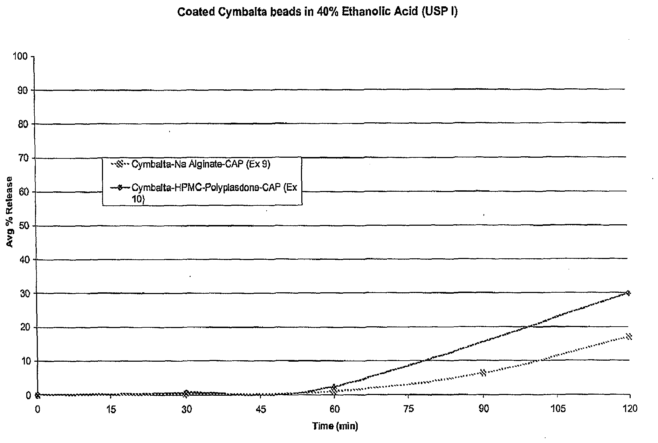

Fig. 3 is a plot of the released amount of drug, duloxetine (% released) over time (min) in 40% ethanolic acid of Cymbalta® beads coated with aqueous sodium alginate and organic-based CAP dispersion (Example 9) and Cymbalta® beads coated with aqueous HPMC/Polyplasdone® XL and organic-based CAP dispersion (Example 10).

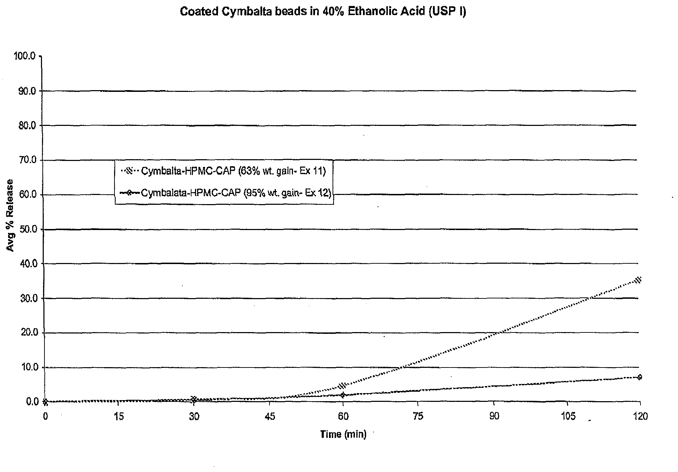

Fig. 4 is a plot of the released amount of drug, duloxetine (% released) over time (min) in 40% ethanolic acid of Cymbalta® beads coated with aqueous HPMC and organic-based CAP dispersion (Example 11) and Cymbalta® beads coated with aqueous HPMC and organic-based CAP dispersion (Example 12).

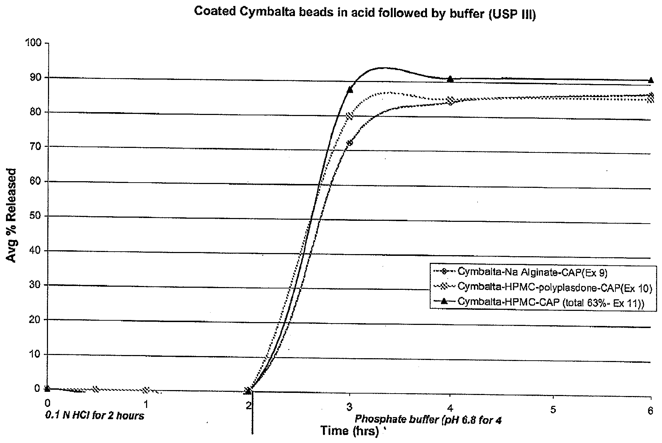

Fig. 5 is a plot of the released amount of drug, duloxetine (% released) over time (min) of the following samples in 0.1N HCl (2 hrs) and phosphate buffer (pH 6.8, 4 hrs) in USP III (1) Cymbalta® beads coated with aqueous sodium alginate and organic-based CAP dispersion (Example 9); (2) Cymbalta® beads coated with aqueous HPMC/Polyplasdone® XL and organic-based CAP dispersion (Example 10); and (3) Cymbalta® beads coated with aqueous HPMC and organic-based CAP dispersion (Example 11);

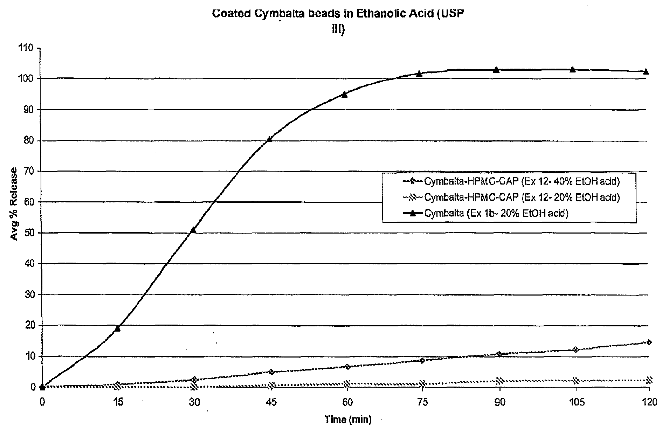

Fig. 6 is a plot of (1) uncoated, commercially available Cymbalta® beads in 20% Ethanolic acid in USP III (Example 1b); (2) Cymbalta® beads coated with aqueous HPMC and organic-based CAP dispersion in 20% Ethanolic acid in USP III (Example 12); (3) Cymbalta® beads coated with aqueous HPMC and organic-based CAP dispersion in 40% Ethanolic acid in USP III (Example 12).

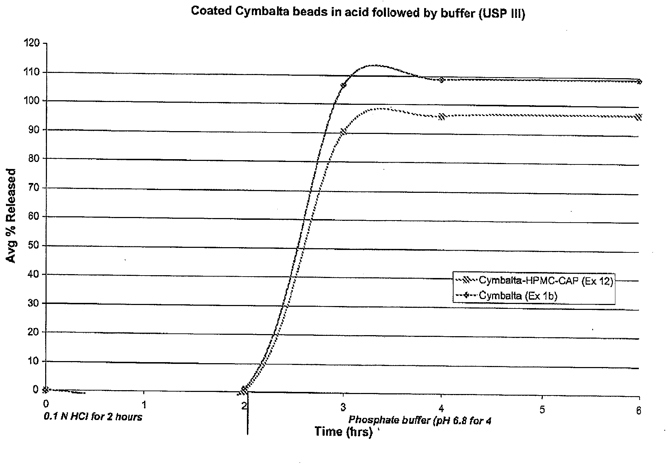

Fig 7 is a plot of the released amount of drug, duloxetine (% released) over time (min) of the following samples in 0.1N HCl (2 hrs) and phosphate buffer (pH 6.8, 4 hrs) in USP III (1) uncoated, commercially available Cymbalta® beads (Example 1); and (2) Cymbalta® beads coated with aqueous HPMC and organic-based CAP dispersion (Example 12)
Fig. 8 is a plot of the % release of duloxetine in 0.1 N HCl/40% ethanolic acid (2 hours) followed by phosphate buffer (4 hours) of the formulation described in Examples 12.
Fig. 9 is a plot of the % release of fenofibric acid in ethanolic phosphate (pH 3.5) for 2 hours followed by phosphate buffer (pH 6.8) of TriLipix® as described in more detail at Example 13.
Fig. 10 is a plot of the % release of fenofibric acid in ethanolic phosphate (pH 3.5) for 2 hours followed by phosphate buffer (pH 6.8) of a formulation of TriLipix® coated according to an embodiment of the invention as described in more detail at Example 13.
Fig. 11 is a plot of the % release of esomeprazole magnesium from NEXIUM® beads in 0.1N HCl/40% ethanolic acid (2 hours) followed by phosphate buffer (4 hours) of the formulation described in Examples 13.
Fig. 12 is a plot of the % release of esomeprazole magnesium from NEXIUM® beads coated with 63% and 77% CAP in 0.1N HCl/40% ethanolic acid (2 hours) followed by phosphate buffer (4 hours).
Fig. 13 is plot of the % release of esomeprazole magnesium from NEXIUM® beads and CAP coated NEXIUM® beads in 0.1 NHCl followed by phosphate buffer (4 hours).
Fig. 14 is plot of the % release of esomeprazole magnesium from NEXIUM® coated with 30% Eudragit S in 0.1N HCl/40% ethanolic acid (2 hours) followed by phosphate buffer (4 hours).

### DETAILED DESCRIPTION OF THE INVENTION

The FDA has indicated that for controlled release dosage forms, in vitro testing for alcohol-induced dose dumping may be advisable as a routine characterization test. Not only would these test be relative to opioids, such a hydormorphone and morphine, it would be recommended for certain other drugs, for example but not limited to, drugs with a narrow therapeutic index or drugs that if dose dumped result in dire consequences of high Cₘₐₓ or low Cₘᵢₙ or drugs that if dumped would result in adverse toxicological events. FDA prefers that formulations be made ethanol-resistant by design, rather than simply a confirmation that dose dumping does not occur through an in vivo study. (cf. Summary of FDA's position on alcohol-induced dose dumping as presented at the Pharmaceutical Sciences Advisory Committee Meeting Oct. 26, 2005).

The FDA has suggested conducting the in-vitro dissolution testing of the controlled release dosage forms for two hours in varying concentrations of Ethanolic HCl (0.1N), such as 5% Ethanolic HCl (0.1N), 20% Ethanolic HCl (0.1N), and 40% Ethanolic HCl (0.1N) sampling every 15 minutes when appropriate followed by a phosphate buffer bath at pH 6.8 for four (4) hours. Bath conditions are determined appropriately based upon the dosage form, and include U.S. Pharmacopeia Apparatus (USP) I (basket, 40 mesh) paddle speed 75 rpm (media volume: 900 mL @ 37°C) with a weight based equivalent of 60 mg of active agent or USP III (40 mesh) media volume 250 mL 37°C with a weight based equivalent of 15 mg of active agent. (See *Dissolution Testing: An FDA Perspective,* AAPS Workshop, Physical Pharmacy and Biopharmaceutics, Division of Bioequivalence-2, Office of Generic Drugs, CDER/FDA, 13 May 2009) Such a test was used to study the pharmaceutical formulations of the present invention. As of the 2009 AAPS Workshop, the FDA does not request dissolution profiles in multimedia for DR products.

In one aspect, the present invention is directed to those active agents that should not be allowed to dissolve in the stomach, e.g. because they are not absorbed, or they may undergo acid degradation or they may irritate the stomach, but are dissolved when the dosage form reaches a more neutral pH, such as that of the lower or small intestine. Typically, these active agents would require a pharmaceutical formulation that prevents dissolution in the stomach - commonly referred to as enteric formulations ("EC") or delayed release ("DR") formulations. In contrast to these formulations are other formulations referred to as "extended release ER or XR," "controlled release CR," "once-daily", or "once-a-day" products (see e.g., COREG® CR (once-a-day carvedilol phosphate, GlaxoSmithKline) and ADDERALL® XR, (amphetamine, dextroamphetamine mixed salts, Shire US Inc.)). These non-enteric formulations are specifically designed to release a portion of the active agent in the stomach as well as release active agent in the small intestines in a controlled manner. Notwithstanding whether the product is called "controlled release," "extended release," "once-daily", or "once-a-day" for the purposes on this invention, the critical determination is whether the pharmaceutical formulation does or does not allow the release of the active agent in the stomach. According to one exemplary embodiment, the present invention is directed to those active agents that should not be allowed to significantly dissolve in the stomach.

The term "dumping" as used herein describes either a catastrophic release of the active or a release which would not be bioequivalent according to FDA standards for Cₘₐₓ, Tₘₐₓ and/or AUC parameters. The United States Food and Drug Administration (FDA) has defined bioequivalence as, "the absence of a significant difference in the rate and extent to which the active agent or active moiety in pharmaceutical equivalents or pharmaceutical alternatives becomes available at the site of drug action when administered at the same molar dose under similar conditions in an appropriately designed study." (FDA, 2003) In other words, the FDA considers two products bioequivalent if the 90% CI of each or all the relative mean Cₘₐₓ, AUC₍₀₋ₜ₎ and AUC_{(0-∞)} of the test formulation to reference formulation should be within 80.00% to 125.00%.

When bioequivalency studies cannot be completed because it would put the subject harms way, an in vitro dissolution test of the test formulation is compared to a reference formulation (e.g., a commercially equivalent product). This is an FDA acceptable determination of whether the test formulation (e.g., the alcohol protected formulation of the present invention) is equivalent to the reference formulation (e.g., a commercially equivalent product). When comparing the test and reference formulations, dissolution profiles should be compared using a similarity factor (f2). The similarity factor is a logarithmic reciprocal square root transformation of the sum of squared error and is a measurement of the similarity in the percent (%) of dissolution between the two curves. Two dissolution profiles are considered "similar" when the f2 value is ≥50. See *Waiver of In Vivo Bioavailability and Bioequivalence Studies for Immediate-Release Solid Oral Dosage Forms Based on a Biopharmaceutics Classification System,* U.S. Department of Health and Human Services, Food and Drug Administration Center for Drug Evaluation and Research (CDER), August 2000.

There are a number of known formulations to prevent release of the active agent from the formulation as it passes through the stomach. Examples include those formulations discussed in U.S. Patent Nos. 7,011,847; 6,159,501; 5,273,760; and U.S. Published Patent Applns. 2008/0085304; 2004/0170688; and 2008/0226711.

Materials used in these systems include, for example, fatty acids, waxes, shellac and plastics. Typically, the materials that make of such systems are segregated into two groups: aqueous-based and solvent-based systems. Most enteric systems work by presenting a surface that is stable at the highly acidic pH found in the stomach, but breaks down rapidly at a less acidic (relatively more basic) pH. For example, the enteric systems will not dissolve in the acidic juices of the stomach (about pH 3), but they will dissolve in the higher pH (approx. above pH 5, such as 5.5) environment present in the small intestine.

Any system that prevents dissolution of the active agent in the stomach, including but not limited to those exemplified above, are herein referred to collectively as "enteric systems." Non-limiting examples of enteric systems include aqueous and organic based HPMC-AS: hydroxyl propyl methyl cellulose acetate succinate -HF (AQOAT sold by Shin-Etsu Chemical Co., Ltd. of Japan); PVAP: poly vinyl acetate phthalate (SURETERIC® by Colorcon, Inc., Harleysville, PA); aqueous-based CAP: cellulose acetate phthalate (AQUACOAT®-CPD by FMC Biopolymer of Philadelphia, PA); organic based CAP: cellulose acetate phthalate (Eastman C-A-P, Eastman Co.); poly(methacylic acid-co-ethyl acrylate) anionic copolymers sold under the tradename EUDRAGIT® grade L, S, and FS (Evonik Degussa, Darmstadt, DE).

The enteric system is applied to the dosage form as a layer or coating, or is in the form of a matrix. The enteric system is a single material, or a combination of materials.

Exemplary commercially available pharmaceutical formulations that employ an enteric system in the form of a coating or layer to prevent the active agent from dissolving in the stomach include CYMBALTA® (duloxetine HCl, Lilly USA, LLC); NEXIUM® (esomeprazole, AstraZeneca LP); ACIPHEX® (rabeprazole sodium, Eisai Inc. and Ortho-McNeil-Janssen Pharmaceuticals, Inc.); ASACOL® HD (mesalamine, Procter & Gamble Pharmaceuticals, Inc.); LIALDA® (mesalamine, Shire US Inc.); PENTASA® (mesalamine, Shire US Inc); ENTECORT® EC (budesonide capsules, AstraZeneca LP); LAMICTAL® XR (lamotrigine tablets, GlaxoSmithKline); KAPIDEX® (dexlansoprazole, Takeda Pharmaceuticals North America, Inc.); Creon® (pancreatin capsules, Solvay S.A); ULTRASE® (pancrelipase capsules, Axcan Pharma US); PROTONIX® (pantoprazole, Pfizer Inc.); DEPAKOTE® (divalproex sodium, Abbott Laboratories); PROLOSEC® (omeprazole, AstraZeneca LP); PREVACID® (lanzoprazole, Novartis Consumer Health, Inc.); ARTHOTEC® (diclofenac sodium, Pfizer Inc.); STAVZOR® (valproic acid, Noven Therapeutics LLC); TRILIPIX® (fenofibric acid delayed release capsules, Abbott Laboratories); and VIDEX® EC (didanosine, Bristol-Myers Squibb).

Exemplary active agents (whether available in commercially sold products or not) that employ or may employ an enteric layer to prevent the active agent from dissolving in the stomach include aspirin, bisacodyl, naproxen, erythromycin, sodium rabeprazole, adenovirus vaccine type 4, calcitonin, darapladib, mesalzine, alendronic acid, eprotirome, NE-F (Nephritic factor), glatiramer, CH-1504 (a non-metabolized antifolate from Chelsea Therapeutics International, Ltd.), ORAZOL® (bisphosphonate (zoledronic acid) compound, Merrion Pharmaceuticals), mercaptamine, larazotide, and oral insulin.

The present invention is not limited to the currently commercialized enteric dosage forms and is contemplated to be used with an active agent that is susceptible to ethanol-induced dumping.

An exemplary embodiment of the alcohol-resistant pharmaceutical composition of the present invention utilizes an "alcohol protectant" to prevent or retard ethanol-induced dumping of the active agent from the dosage form.

The alcohol protectant may be a single material, e.g. a polymer, or a combination of materials, e.g., a combination of polymers in an excipient solution. The alcohol protectant is deposited in layer or coating, or it is in the form of a matrix in alternative embodiments. Suitable alcohol protectant materials include, but are not limited, to organic based cellulose acetate phthalate, ammonium methacrylate copolymers, methacrylate ester copolymers, methacrylic acid copolymers, natural and synthetic starches, polyalkylene oxides, and natural and synthetic celluloses including modified celluloses such as hydroxypropylmethylcellulose (HPMC), hydroxypropylcellulose (HPC) hydroxymethylcellulose (HMC), methylcellulose (MC), hydroxyethylcellulose (HEC), and carboxymethylcellulose (CMC), waxes such as insect and animal waxes, vegetable waxes, mineral waxes, petroleum waxes, and synthetic waxes.

The present compositions include organic based cellulose acetate phthalate as an alcohol protectant. In an exemplary embodiment, the alcohol protectant is an organic based cellulose acetate phthalate sold under the trade name Eastman C-A-P® or Cellacefate, NF by the Eastman Chemical Company, Kingsport, TN USA.

The alcohol protectant may be present in the formulation in an amount sufficient to impart alcohol resistance at a given ethanolic concentration. According to one aspect of the invention, the alcohol protectant is add to a commercially equivalent formulation in an amount of 20%, 25%, 30%, 35%, 40%, 45%, 50%, 55%, 60%, 65%, 70%, 75%, 80%, 85%, 90%, 95% 100%, 150%, 200%, 250%, 300%, 350%, 400%, 450% and 500% by weight gain.

The pharmaceutical composition of the present invention is alcohol resistant based upon a relationship between the percentage release of active agent from the dosage form in an alcohol environment, or in an non-alcohol environment after the dosage form was exposed to an alcohol environment. In other exemplary embodiments, the present invention is an alcohol-resistant pharmaceutical composition that provides resistance to ethanol-induced dumping and is bioequivalent to the commercially equivalent formulation of the active agent.

As discussed previously, in order to quantify the resistance to ethanol-induced dumping, a dissolution test was performed in 5%, 20%, and 40% ethanolic HCl (see FDA Guidelines discussed above) for two hours. Applicants added ethanolic concentrations at 30% and 35% as well.

In another experiment to quantify the resistance to ethanol-induced dumping, two, separate dissolution tests were performed, one in 0.1N HCl (2 hours, as described above), then another (using a different sample) in phosphate buffer pH 6.8 (4 hours). The dissolution profiles of each were then analyzed.

In yet another experimental design to quantify the resistance to ethanol-induced dumping, sequential dissolution of the same sample was performed. This dissolution test involved dissolution in ethanolic acid (2 hours) followed by phosphate buffer pH 6.8 (4 hours). The sequential ethanolic acid and phosphate buffer baths are intended to mimic in vivo conditions of a person imbibing alcohol concomitantly with the administration of the dosage form. The dosage form that would first pass through the alcoholic/acidic stomach (average gastrointestinal residence time ∼ 2 hrs) and then pass through into the small intestines, which are at a more neutral pH (average gastrointestinal residence time ∼ 4 hrs). Ethanol is not believed to be in the lower intestine as is it rapidly absorbed in the stomach.

Dissolution studies were performed using USP Apparatus I (Baskets, 40 mesh) @ 75rpm [Media Volume: 900mL @ 37°C] with a 60 mg weight equivalent of active; and USP Apparatus III (40 mesh) [Media Volume: 250 mL @ 37°C] with a 15 mg weight equivalent of active.

One would not want the enteric coat of a formulation containing an active agent known to form toxic degradents in the stomach to fail when exposed to an alcohol environment. One such product that suffers this fate is CYMBALTA® (enteric coated duloxetine HCl) sold by Lilly, Inc. As reported in The Rearrangement of Duloxetine Under Mineral Acid Conditions, RJ Bopp, AP Breau, TJ Faulkinbury, PC Heath, C Miller, 206th Natl. Am. Che. M. Soc. Meeting; Mar 13 1993, Abstract# 111; duloxetine HCl rapidly undergoes solvolysis and rearrangement in aqueous HCl to yield a 1-(2-thieayl)carbinol, naphthol, and a 1-(2-thienyl) 2- and 4-substituted naphthols.

Now consider an enteric-coated formulation containing an active which is not known to cause toxic effects if allowed to dissolve or even dose-dump in the stomach, but rather the consequence of dose dumping is a sub-therapeutic effect of the active. One such example of this is TriLipix® (fenofibric acid also referred to as choline fenofibrate), manufactured by Abbott Laboratories of North Chicago, IL. Abbot conducted a series of studies demonstrating that fenofibric acid immediate release tablets had a significantly higher (1.4 fold) Cmax, a lower (0.67 fold) Tmax, and a fed/fasted variability compared to Tricor®-145 (fenofibrate). Their regiospecific study led to the conclusion that in order to develop a formulation bioequivalent to the commercially available fenofibrate tablet, the release profile of the formulation containing fenofibric acid (i.e., TriLipix®) needed to be slowed in order to match the slower absorption properties of fenofibrate (Tricor®-145) in the GI tract. See TriLipix® SBA Study K LF178P 03 03 KH 05 02 (regiospecific study) page 43. With this in mind, according to the Summary Basis of Approval, the TriLipix® Medical Review Table 7.2.1.D. Demographics and Baseline Characteristics for Study M05-758 identified 52.3% of the target patient population of TriLipix® as "Drinkers," 7.2% as "Ex-Drinkers," as 40.5% were "non-drinkers." Thus, should the fenofibric acid of Trilipix® be allowed to release in the stomach as a consequence of ethanol-induced dumping, it would result in a higher Cmax and shorter Tmax of the active ingredient.

In one embodiment, the present invention prevents or retards ethanol-induced dumping of the active agent of the formulation to the degree where no measurable active agent is released when the dosage form is placed in 40% ethanol. Accordingly, the alcohol protectant imparts resistance to ethanol-induced dumping when not more than 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99%, of the active is released from the dosage form in 40% ethanol after 0.25, 0.5, 0.75, 1, 1.25, 1.5, 1.75, or 2 hrs. Additionally, the alcohol protectant imparts resistance to ethanol-induced dumping when not more than about 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99% of the active is released from the dosage form in 35% ethanol after 0.25, 0.5, 0.75, 1, 1.25, 1.5, 1.75, or 2 hrs. Yet additionally, the alcohol protectant imparts resistance to ethanol-induced dumping when not more than about 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99% of the active is released from the dosage form in 30% ethanol after 0.25, 0.5, 0.75, 1, 1.25, 1.5, 1.75, or 2 hrs. Still yet, the alcohol protectant imparts resistance to ethanol-induced dumping when not more than about 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99% of the active is released from the dosage form in 20% ethanol after 0.25, 0.5, 0.75, 1, 1.25, 1.5, 1.75, or 2 hrs. Still further yet, the alcohol protectant imparts resistance to ethanol-induced dumping when not more than about 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99% of the active is released from the dosage form in 5% ethanol after 0.25, 0.5, 0.75, 1, 1.25, 1.5, 1.75, or 2 hrs.

In another embodiment, the invention is directed to a formulation that prevents or retards ethanol-induced dumping of the active agent where the amount of the active agent released is less than the amount of active agent released from a commercially equivalent formulation. By "commercially equivalent formulation or product" it is understood to mean that formulation of the active agent which is approved for use by the FDA, but which does not have the alcohol protectant feature of the present invention. For example, according to this embodiment, the invention is directed to a formulation where an amount of active agent is released in the presence of alcohol, but that amount is less than the amount released by the commercially equivalent formulation.

Accordingly, the alcohol protectant imparts resistance to ethanol-induced dumping when not more than 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99%, of the active is released from the dosage form in 40% ethanol after 0.25, 0.5, 0.75, 1, 1.25, 1.5, 1.75, or 2 hrs when compared to the amount of active agent released by the commercially equivalent formulation in the same concentration of ethanol for the same time. Additionally, the alcohol protectant imparts resistance to ethanol-induced dumping when not more than about 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99% of the active is released from the dosage form in 35% ethanol after 0.25, 0.5, 0.75, 1, 1.25, 1.5, 1.75, or 2 hrs when compared to the amount of active agent released by the commercially equivalent formulation in the same concentration of ethanol for the same time. Yet additionally, the alcohol protectant imparts resistance to ethanol-induced dumping when not more than about 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99% of the active is released from the dosage form in 30% ethanol after 0.25, 0.5, 0.75, 1, 1.25, 1.5, 1.75, or 2 hrs when compared to the amount of active agent released by the commercially equivalent formulation in the same concentration of ethanol for the same time. Still yet, the alcohol protectant imparts resistance to ethanol-induced dumping when not more than about 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99% of the active is released from the dosage form in 20% ethanol after 0.25, 0.5, 0.75, 1, 1.25, 1.5, 1.75, or 2 hrs when compared to the amount of active agent released by the commercially equivalent formulation in the same concentration of ethanol for the same time. Still further yet, the alcohol protectant imparts resistance to ethanol-induced dumping when not more than about 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99% of the active is released from the dosage form in 5% ethanol after 0.25, 0.5, 0.75, 1, 1.25, 1.5, 1.75, or 2 hrs when compared to the amount of active agent released by the commercially equivalent formulation in the same concentration of ethanol for the same time.

In another aspect, the invention is related to formulations that do not dose dump in an alcohol environment, and when subsequently placed into a phosphate buffer (to simulate the digestive track changes in pH downstream of the stomach) have substantially the same release profile when compared to the same formulation in phosphate buffer dissolution, where the formulation has not undergone previous exposure to ethanolic acid. In this aspect of the invention, the formulation of the invention has a release rate in phosphate buffer that is not substantially affected by the previous exposure to an alcohol environment. Table 2 shows some commercially available dosage forms (i.e., commercially equivalent dosage forms) that appear to be robust in an ethanolic acid environment, but when subsequently tested in phosphate buffer, show a change in their dissolution rate.

**Table 2**

| **Drug Product** | **Single Stage Media (0-2) hr in 0.1 N HCl and 40%** | **2 Stage Media (0 - 2) hr in 0.1N HCl, 40% Alcohol (2 - 4h) r in Phosphate Buffer, pH** | **Coating (& inactive ingredients)** |
|---|---|---|---|
| Aciphex DR Tablets (Rabeprazole sodium) | No peaks observed | Drug released 10min earlier after 40% Alcohol treatment than in 0.1N HCl alone. | Sugar spheres, magnesium carbonate, sucrose, low-substituted hydroxypropyl cellulose, titanium dioxide, hydroxypropyl cellulose, hypromellose 2910, talc, methacrylic acid copolymer, polyethylene glycol 8000, triethyl citrate, polysorbate 80, and colloidal silicon dioxide. Bead 1: Eudragit L30 D-55 or Eudragit L100-55 Bead 2: Blend of Eudragit S100 and Eudragit L-100 |
| Kapidex DR Capsules (Dexlansoprazol e) | No peaks observed | Significant difference in drug release rate. | Colloidal silicon dioxide; crospovidone; hydrogenated castor oil; hypromellose; lactose; magnesium stearate; methacrylic acid copolymer; microcrystalline cellulose; povidone (polyvidone) K-30; sodium hydroxide; starch (corn); talc; triethyl citrate. |

According to this embodiment of the invention, the formulation of the invention do not dose dump in an alcohol environment, and when subsequently placed into a phosphate buffer, demonstrates substantially the same in vivo bioequivalent pharmacokinetic profile and/or similar in vitro dissolution profile when compared to the same formulation in phosphate buffer, but which has not been previously exposed to an alcohol environment.

Accordingly, the alcohol protectant imparts resistance to ethanol-induced dumping when, after 2 hours in ethanolic acid (40% ethanol in 0.1N HCl), no measureable active agent is released and the difference between the amount of active agent released by the alcohol protected formulation of the invention and that amount released by the commercially equivalent formulation when both formulations are subsequently placed in phosphate buffer pH 6.8 (4 hours) is 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99%. The alcohol protectant imparts resistance to ethanol-induced dumping when, after 2 hours in ethanolic acid (35% ethanol in 0.1N HCl), no measureable active agent is released and the difference between the amount of active agent released by the alcohol protected formulation of the invention and that amount released by the commercially equivalent formulation when both formulations are subsequently placed in phosphate buffer pH 6.8 (4 hours) is 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99%. The alcohol protectant imparts resistance to ethanol-induced dumping when, after 2 hours in ethanolic acid (30% ethanol in 0.1N HCl), no measureable active agent is released and the difference between the amount of active agent released by the alcohol protected formulation of the invention and that amount released by the commercially equivalent formulation when both formulations are subsequently placed in phosphate buffer pH 6.8 (4 hours) is 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99%. The alcohol protectant imparts resistance to ethanol-induced dumping when, after 2 hours in ethanolic acid (20% ethanol in 0.1N HCl), no measureable active agent is released and the difference between the amount of active agent released by the alcohol protected formulation of the invention and that amount released by the commercially equivalent formulation when both formulations are subsequently placed in phosphate buffer pH 6.8 (4 hours) is 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99%. The alcohol protectant imparts resistance to ethanol-induced dumping when, after 2 hours in ethanolic acid (5% ethanol in 0.1N HCl), no measureable active agent is released and the difference between the amount of active agent released by the alcohol protected formulation of the invention and that amount released by the commercially equivalent formulation when both formulations are subsequently placed in phosphate buffer pH 6.8 (4 hours) is 1%, 2%, 5%, 8%, 10%, 15%, 20%, 25%, 30%, 35%, 40%, 45%, 50%, 55%, 60%, 65%, 70%, 75%, 80%, 85%, 90%, 95%, or 99%.

According to one exemplary embodiment of the alcohol-resistant pharmaceutical composition of the invention where the dosage form is a multiparticulate, the alcohol protectant is applied as a layer or coating during the manufacturing of the dosage form. It is not important that the coating or layer formed with alcohol protectant may have slight or microscopic gaps, cracks, crevices, or holes. Rather, the critical feature is whether the coating or layer imparts the formulation with resistance to ethanol-induced dose dumping.

In the embodiment where the alcohol protectant is a layer or coating, the alcohol protectant is exterior to the active agent, whether that active agent is part of a core, layer or dispersed within a matrix. For example, in one embodiment, the alcohol protectant may be applied as a coating directly to the active agent in bulk form. For example, typical bulk drug has a particle size greater than 10 µm. These bulk drug particles may be directly coated with the alcohol protectant and then compressed into a tablet, which tablet receives an enteric coat. Alternatively, the alcohol-protected coated drug particles may be placed within a matrix, which is made from an enteric material, or which matrix is itself coated with an enteric coat. In a further embodiment, the material that comprises the alcohol protectant is not a layer or coating, but is co-mixed, admixed, commingled with or blended with the active agent within the dosage form.

In some embodiments, the ability to prevent the active from dose dumping in the presence of alcohol and the ability to prevent the active from dissolving in the acidic environment of the stomach are embodied in a combination of materials or polymers combined in an excipient mixture or embodied in a single polymer system and disposed in a layer, coating or formed into a matrix. For the purposes herein, it is understood that when referring to the alcohol protectant, it is envisage that it may have enteric properties. Likewise, it is understood that when referring to the enteric material, it is envisage that it may retard ethanol induced dose dumping.

In the embodiment where the dosage form is a multiparticulate bead, to apply the alcohol layer onto a multiparticulate bead, the beads (30g to 50g) were coated using fluidised bed coater (Mini Vector, MFL 01).

The amount of alcohol protectant (and disintegrant discussed below) included in the alcohol-resistant pharmaceutical composition of the present invention is determined by a percentage weight gain. For example, in the embodiment where the dosage form is a multiparticulate bead, the bead to be coated weighs 10 gm and a 10% by weight layer of alcohol protectant is to be coated thereon, then a sufficient amount of alcohol protectant layer is sprayed onto the bead so that the total weight of the bead would increase to 11 gms. Mathematically, (1gm of added alcohol protectant/10gm original bead weight)*100%=10% weight gain). In another example, if one desires to add a disintegrant (discussed in more detail below) to a bead with a 20% weight gain, then one would spray enough disintegrant material onto the bead in a layer or coating to add 2 gms of weight to the bead. If one wants to add the alcohol protectant onto this bead (which now has a total weight of 12 gm) at a 50% weight gain, one would spray a sufficient amount of alcohol protectant material to bring the total weight of the bead to 18 gm ((6gm of alcohol protectant material / 12 gm bead)*100% is 50% weight gain).

The alcohol protectant material is present in the dosage form in an amount that provides a percentage weight gain ranging from 10% to 500%, 20% to 80%, 30% to 70%, 40% to 60%, or 45% to 55%. Alternatively the alcohol protectant material is present in the dosage form in an amount that provides a percentage weight gain of about 20%, 25%, 30%, 35%, 40%, 45%, 50%, 55%, 60%, 65%, 70%, 75%, or 80%.

In a further embodiment, the present invention includes a disintegrant which is comprised of a swellable material and/or a superdisintegrant.

Exemplary swellable materials include, but are not limited to, agar, alginic acid, carbomers, carregeenan, cellulose acetate, chitosan, guar gum, hydroxypropyl cellulose, hypromellose, hypromellose acetate succinate, hypromellose phthalate, methyl cellulose, poloxamer, polycarbophil, polyethylene oxide, povidone, sodium hyaluronate, xanthan gum, and zein. The swellable material present in the disintegrant is in an amount of from about 1%, 2%, 3%, 5%, 7%, 9%, 10%, 12%, 14%, 15%, 17%, 19%, 20%, 22%, 23%, 24%, 25%, 27%, 29%, 30%, 32%, 35%, 38%, 40%, 45%, 50%, 55%, 60%, 65%, 70%, 75%, 80, 85%, 90%, 95%, 98%, 99%, or 100% (when the disintegrant is all swellable material) .

Exemplary superdisintegrants include, but are not limited to Polyplasdone® XL or XL-10 (1-ethenylpyrrolidin-2-one, ISP Pharmaceuticalsis, Columbia, MD); calcium alginate, carboxymethylcellulose calcium, carboxymethylcellulose sodium, cellulose, chitosan, colloidal silicon dioxide, croscarmellose sodium, crospovidone, docusate sodium, guar gum, hydroxypropyl cellulose, magnesium aluminium silicate, methylcellulose, microcrystalline cellulose, polarcrillin potassium, povidone, sodium alginate, sodium starch glcolate, and starch. The superdisintegrant is present in the disintegrant is in an amount of from about 1%, 2%, 3%, 5%, 7%, 9%, 10%, 12%, 14%, 15%, 17%, 19%, 20%, 22%, 23%, 24%, 25%, 27%, 29%, 30%, 32%, 35%, 38%, 40%, 45%, 50%, 55%, 60%, 65%, 70%, 75%, 80, 85%, 90%, 95%, 98%, 99%, or 100% (when the disintegrant is all superdisintegrant).

The disintegrant (whether comprised solely of superdisintegrant or a combination of superdisintegrant and swellable material) is present in the dosage form in an amount that provides a percentage weight gain ranging from about 20% to 80%, 30% to 70%, 40% to 60%, or 45% to 55%. Alternatively the disintegrant is present in the dosage form in an amount that provides a percentage weight gain of 20%, 25%, 30%, 35%, 40%, 45%, 50%, 55%, 60%, 65%, 70%, 75%, or 80%.

Under certain circumstances, the alcohol protectant may interact with the active agent an effect the dissolution/release of the active. Accordingly, in yet another embodiment, the alcohol-resistant pharmaceutical composition includes a barrier material disposed between the active agent and the alcohol protectant.

### EXAMPLES

The following examples are given to illustrate the present invention. It should be understood, however, that the invention is not to be limited to the specific conditions or details described in these examples. Experiments listed below as Examples 1a, 1b, 2a, 2b, 2c, and 3-5 are provided as reference examples.

Table 1 tabulates the studies conducted on the commercially available Cymbalta® duloxetine HCL immediate release capsules.

| **Table 1** | Type of | Coating gain | % weight | % release in 20% ethanolic acid (2 hrs)* | % release in 40% ethanolic acid (2 hrs)* | % release in 0.1N HCl (2 hrs.)* | % release in phosphate buffer (4 hrs)* |
|---|---|---|---|---|---|---|---|
| **Example 1a** | Cymbalta ® beads | NONE | N/A | 80 (USP I) | >98 (USP I) | No measurable drug | >99 (USP I) |
| **1b** | Cymbalta ® beads | NONE | N/A | >99 (USP III) | Not Tested | No measurable drug | >99 (USP III) |

### EXAMPLE 1 (a and b)

Commercially available Cymbalta® (duloxetine HCl) 60 mg, delayed release capsules (referred to herein as "Cymbalta® beads") released 80% drug at 2 hrs in 20% ethanolic acid (USP I) and substantially all the drug was released at 2 hrs in 40% Ethanolic acid (USP I). Cymbalta® beads released substantially all the drug at 2 hrs in 20 % ethanolic acid while using USP III.

Further dissolution testing was conducted in 0.1N HCl (two hours, USP I and USP III) followed by phosphate buffer (pH 6.8, 4 hours, USP I and USP III). No measurable drug was released in the acid and substantially all the drug was released in phosphate buffer after 4 hours.

**Table 2 tabulates the results described in Examples 2-5.**

| **Table 2** | Type of bead | Coating | % weight gain | Targeted release in 20% ethanolic acid (2 hrs)* | % release in 40% ethanolic acid (2 hrs)* |
|---|---|---|---|---|---|
| **Example 2a** | Cymbalta® beads | aqueous hydroxyl propyl methyl cellulose acetate succinate-HF | 15 | 60 | >99 |
| **2b** | Cymbalta® beads | aqueous poly vinyl acetate phthalate | 15 | Not Tested | >90 |

| **Table 2** | Type of bead | Coating | Targeted % weight gain | % release in 20% ethanolic acid (2 hrs)* | % release in 40% ethanolic acid (2 hrs)* |
|---|---|---|---|---|---|
| **2c** | Cymbalta® beads | aqueous CAP | 10 and 50 | Not Tested | >99 |
| | | | | | >99 |
| **Example 3** | Cymbalta® beads | organic-based ethyl acrylate, methyl methacrylate polymers50:50 ratio | 40 | <20 | >99 |
| **Example 4** | Duloxetine IR beads | organic-based ethyl acrylate, methyl methacrylate polymers 50:50 ratio | 30 | >90 | Not Tested |
| | Duloxetine IR beads | organic-based ethyl acrylate, methyl methacrylate polymers 40:60 ratio | 30 | >99 | Not Tested |
| | Duloxetine IR beads | organic-based ethyl acrylate, methyl methacrylate polymers 60:40 ratio | 30 | >99 | Not Tested |
| **Example 5** | Cymbalta® beads | organic-based ethyl acrylate, methyl methacrylate polymers 50:50 ratio filled in V-Caps® | 50 | 4 | 39 |

### EXAMPLE 2 (a, b, and c)

Cymbalta® coated with aqueous based enteric dispersions such as Hydroxyl Propyl Methyl Cellulose Acetate Succinate -HF (AQOAT sold by Shin-Etsu Chemical Co., Ltd. of Japan), Poly Vinyl Acetate Phthalate (SURETERIC® by Colorcon, Inc., Harleysville, PA) and aqueous-based Cellulose Acetate Phthalate (AQUACOAT®-CPD by FMC Biopolymer of Philadelphia, PA) released substantially all the drug at 2 hrs in 40% ethanolic acid.

### EXAMPLE 3

Cymbalta® beads coated with Eudragit® RS and Eudragit® L (50:50) (ethyl acrylate, methyl methacrylate polymers, Evonik Industries, Essen GE) (targeted 40% wt. gain) released less than 20% of drug 2 hrs in 20% ethanolic HCl (USP I) and released substantially all the drug at 2 hrs in 40% ethanolic HCl.

The ethyl acrylate, methyl methacrylate mixture was prepared by dissolving Eudragit® RS polymer in denatured dehydrated alcohol in a low sheer mixer. Eudragit® L polymer was added to the solution until dissolved. Triethyl citrate and talc were added to the solution and mixed until well dispersed. The final composition of the ethyl acrylate, methyl methacrylate mixture that was coated on the Cymbalta® beads is set forth in Table 3.

**Table 3**

| Material | Composition (g) |
|---|---|
| Eudragit® RS PO | 3.5 |
| Eudragit® L 100 55 | 3.5 |
| Triethyl Citrate | 1.4 |
| Talc | 3.5 |
| Denatured Dehydrated Alcohol, USP (SDA-3C) | 83.2 |
| Purified Water | 4.9 |
| Total | 100.0 |

Total Solid content: 11.9% w/w, Dry polymer content: 7.0% w/w

### EXAMPLE 4

Duloxetine immediate release ("IR") beads were manufactured by applying duloxetine dispersion (Table 4) on non-peril sugar beads (Surespheres®, nonpareil spheres 30/35, Colorcon Ltd.) using a fluid bed spray drier (Glatt 1.1).

**Table 4**

| Material | Composition (g) |
|---|---|
| Duloxetine HCl | 7.0 |
| hydroxypropylmethylce llulose | 5.0 |
| Purified Water | 88.0 |
| Total | 100.0 |

Duloxetine IR beads coated with Eudragit® RS and Eudragit® L (ethyl acrylate, methyl methacrylate polymers, Evonik Industries, Essen GE) (50:50, 40:60 and 60:40) (30% - 42% target wt. gain) released substantially all drug at 2 hrs in 20% ethanolic HCl (USP I).

### EXAMPLE 5

Cymbalta® beads coated with Eudragit® RS and Eudragit® L (50:50) (ethyl acrylate, methyl methacrylate polymers, Evonik Industries, Essen GE) (targeted 50% wt. gain) filled in V-caps® (hydroxypropyl methylcellulose two-piece capsules by Capsugel® of Greenwood, SC) released 4% of drug at 2 hrs in 20% ethanolic (USP I) and released 39% drug at 2 hrs in 40% ethanolic acid (USP III) .

| **Table 5** | Type of bead | Coating | % weight gain | % release in 20% ethanolic acid (2 hrs)* | % release in 40% ethanolic acid (2 hrs)* | % rele ase in 0.1N HCl hrs.)* | % release in phosphate buffer (4 hrs)* |
|---|---|---|---|---|---|---|---|
| **Example 6** | Duloxetine IR beads | CAP (Solvent based) | 50 | 25 | Not Tested | 1.5 | 60 |
| **Example 7** | Cymbalta® beads | CAP (solvent based) | 42 | 7 in 35% ethohanolic acid | 36 and 31 (USP I and III, respectively) | No measurable drug release | 65 and 94 (USP I and III, respectively) |

### EXAMPLE 6

Duloxetine IR beads coated with Cellulose Acetate Phthalate (CAP) solvent-dispersion (50% targeted wt gain), released 25% of drug at 2 hrs in 20% ethanolic HCl (USP I). The dissolution was also conducted in 0.1N HCl (two hours, USP I) followed by phosphate buffer (pH 6.8, 4 hours, USP I). At 2 hrs in 0.1N HCl, 1.5% of drug was released. At 4 hrs in phosphate buffer, 60% of drug was released.

The CAP solvent-dispersion was prepared by dissolving CAP in isopropyl alcohol and water. To that solution was added triethyl citrate and talc. The solution was stirred for 12-15 minutes. The final CAP solvent-dispersion composition is set forth in Table 6.

**Table 6**

| Material | Composition (g) |
|---|---|
| Cellulose acetate phthalate (Eastman® CAP) | 8.6 |
| Triethyl Citrate | 1.7 |
| Talc | 1.7 |
| Purified Water | 2.0 |
| Acetone | 43.0 |
| Isopropyl Alcohol (IPA) | 43.0 |
| Total | 100.0 |

Total Solid content: 12% w/w, Dry polymer content: 8.6 % w/w, Plasticizer: 19.77% of polymer

### EXAMPLE 7

Cymbalta® beads coated with CAP solvent-dispersion (42% wt. gain) (as prepared in Example 6) released 7% of drug at 2 hrs in 35% ethanolic (USP I) and 36% of drug at 2 hrs in 40% ethanolic HCl (USP I) (31% when utilising USP III apparatus). Further dissolution testing was conducted in 0.1N HCl (two hours, USP I) followed by phosphate buffer (pH 6.8, 4 hours, USP I). At 2 hrs in acid, no measurable drug was released. At 4 hrs in phosphate buffer, 65% of drug was released (USP I). Utilising USP apparatus III, no measurable drug was released in the acid (0.1N HCl, two hours) and 74% of the drug was released in the phosphate buffer (pH 6.8, 4 hours).

Examples 8-12, tabulated in Table 7, are illustrative of the embodiments of the invention incorporating a disintegrant, which comprises a swellable agent and/or a superdisintegrant.

| **Table** 7 | Type of bead | Coating | % weight gain | % release in 40% ethanolic acid (2 hrs)* | % release in 0.1N HCl (2 hrs.)* | % release in phosphate buffer (4 hrs)* |
|---|---|---|---|---|---|---|
| **Example 8** | Duloxetine IR beads | aqueous HPMC and CAP (solvent based) | Total 84% (24 and 60 respectively) | 70 (USP I) | No measurable drug release | 91 (USP I) |
| **Example 9** | Cymbalta® beads | aqueous sodium alginate and CAP (solvent based) | Total 101% (25 and 75 respectively) | 23 and 17 (USP I and III, respectively) | No measurable drug release | 65 and 87 (USP I and III, respectively) |
| **Example 10** | Cymbalta® beads | aqueous HPMC/ Polyplasdone® XL and CAP (solvent based) | Total 69% (9 and 60 respectively) | 35 and 30 (USP I and III, respectively) | No measurable drug release | 61 and 86 (USP I and III, respectively) |
| **Example 11** | Cymbalta® beads | aqueous HPMC and CAP (solvent based) | Total 63% (20 and 43 respectively) | 36 (USP III) | No measurable drug release | 92 (USP III) |
| **Example 12** | Cymbalta® beads | aqueous HPMC and CAP (solvent based) | Total 95% (20 and 75 respectively) | 15 (2 in 20% ethanolic acid) USP III | No measurable drug release | 97 (USP III) |

### EXAMPLE 8

Duloxetine IR beads coated with aqueous HPMC (24% wt gain) and CAP solvent-dispersion (60% wt gain) (84% total wt gain) (as prepared in Example 6) released 70% of drug at 2 hrs in 40% ethanolic HCl (USP I).

Further dissolution testing was conducted in 0.1N HCl (two hours, USP I) followed by phosphate buffer (pH 6.8, 4 hours, USP I). No measurable drug was released in acid after two hours in HCl. At 4 hrs in phosphate buffer, 91% of drug was released.

The aqueous HPMC coating was prepared by dissolving HPMC and talc in water and mixing for 15-30 minutes until all components were dissolved. The resulting dispersion was filtered through a 150 Micron screen to remove aggregates. The final composition of the aqueous HPMC dispersion is set forth in Table 8.

**Table 8**

| Material | Composition (g) |
|---|---|
| HPMC (Phamacoat® 603) | 5.0 |
| Talc | 7.0 |
| Purified Water | 88.0 |
| Total | 100.0 |

Total Solid content: 12.0% w/w; dry polymer content: 5%, Talc: 140% of polymer

### EXAMPLE 9

Cymbalta® beads coated with aqueous sodium alginate (25 % wt gain) and CAP solvent-dispersion (75% wt gain) (101% total wt gain) (as prepared in Example 6) released 23% of drug at 2 hrs in 40% ethanolic HCl (USP I). A similar dissolution was conducted utilizing USP apparatus III. At 2 hrs in 40% ethanolic HCl, 17% of drug was released.

Further dissolution testing was conducted in 0.1N HCl (two hours, USP I) followed by phosphate buffer (pH 6.8, 4 hours, USP I). At 2 hrs in the acid, no measurable drug was released. At 4 hrs in phosphate buffer, 65% of drug was released. Utilising USP apparatus III, no measurable drug was released in the acid (0.1N HCl, two hours) and 87% of the drug was released in phosphate buffer (pH 6.8, 4 hours).

To prepare the aqueous sodium alginate dispersion, a first solution containing triethyl citrate and talc was prepared in water. Separately, sodium alginate was mixed in a high shear vortex mixer. The sodium alginate was then added to the first solution of triethyl citrate and talc under constant stirring for at least 30 minutes. The final composition of the aqueous sodium alginate dispersion is set forth in Table 9.

**Table 9**

| Material | Composition (g) |
|---|---|
| Sodium Alginate | 0.85 |
| Triethyl Citrate | 0.1 |
| Talc | 0.45 |
| Purified Water | 98.6 |
| Total | 100.0 |

Total Solid content: 1.4% w/w, Dry polymer content: 0.85% w/w; Plasticizer is 11.7% of dry polymer, Talc is 52.9% of dry polymer

### EXAMPLE 10

Cymbalta® beads coated with aqueous HPMC/Polyplasdone® XL (9% wt gain) and CAP solvent-dispersion (60% wt gain) (69% total wt gain) (as prepared in Example 6), released 35% of drug at 2 hrs in 40% ethanolic HCl (USP I). A similar dissolution was conducted utilizing USP apparatus III. At 2 hrs in 40% ethanolic acid, 30% of drug was released (USP III).

Further dissolution testing was conducted in 0.1N HCl (two hours, USP I) followed by phosphate buffer (pH 6.8, 4 hours, USP I). No measurable drug was released in acid. At 4 hrs in phosphate buffer, 61% of drug was released (USP I). Utilising USP apparatus III, no measurable drug was released in the acid (0.1N HCl, two hours) and 86% was released in phosphate buffer (pH 6.8, 4 hours).

To prepare the HPMC/Polyplasdone® XL dispersion a first solution of HPMC was prepared in water. Separately, crospovidone and talc were mixed in a high shear vortex mixer. The crospovidone and talc dispersion was added to the HPMC solution under constant stirring for at least 30 minutes. The final composition of the HPMC/Polyplasdone® XL dispersion is set forth in Table 10.

**Table 10**

| Material | Composition (g) |
|---|---|
| HPMC (Phamacoat 603) | 5.0 |
| Talc | 2.5 |
| Crospovidone (Polyplasdone XL) | 0.5 |
| Purified Water | 92.0 |
| Total | 100.0 |

Total Solid content: 8.0% w/w, Disintegrant content: 0.5% w/w

### EXAMPLE 11

Cymbalta® beads coated with aqueous HPMC (20% wt gain) and CAP solvent-dispersion (43% wt gain) (63% total wt gain) (as prepared in Example 6), released 36% of drug at 2 hrs in 40% ethanolic HCl (USP III).

Further dissolution testing was conducted in 0.1N HCl (two hours, USP III) followed by phosphate buffer (pH 6.8, 4 hours, USP III). At 2 hrs in the acid, no measurable drug was released. At 4 hrs in phosphate buffer, 92% of drug was released (USP III).

### EXAMPLE 12

Cymbalta® beads coated with aqueous HPMC (20% wt gain) and CAP solvent-dispersion (75% wt gain) (95% total wt gain) (as prepared in Example 6), released 15% of drug at 2 hrs in 40% ethanolic HCl (USP III) and 2% of drug at 2 hrs in 20% Ethanolic HCl (USP III). The beads after 40% ethanolic acid study were studied for dissolution in phosphate buffer (pH 6.8, 4 hours, USP III), which released 55 % of drug.

Further dissolution testing was conducted in 0.1N HCl (two hours, USP III) followed by phosphate buffer (pH 6.8, 4 hours, USP III). At 2 hrs in the acid, no measurable drug was released. At 4 hrs in phosphate buffer, 97% of drug was released.

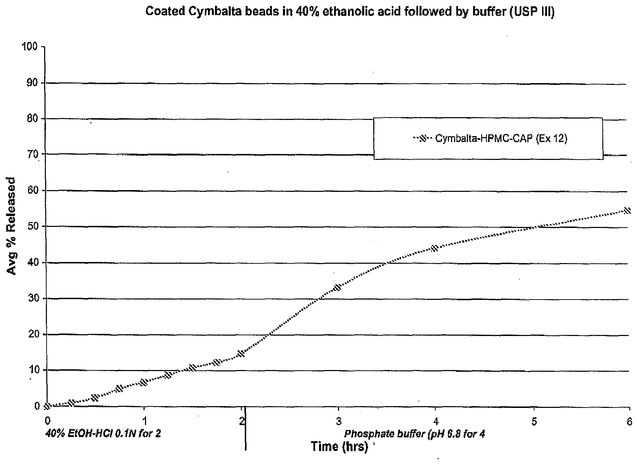

The dissolution characteristics of Example 12 were also studied under slightly different conditions. The composition was placed in 0.1 N HCl/40% ethanolic acid (2 hours) followed by phosphate buffer (4 hours) (USP III). The results of this sequential dissolution test are shown in Fig. 8.

### EXAMPLE 13

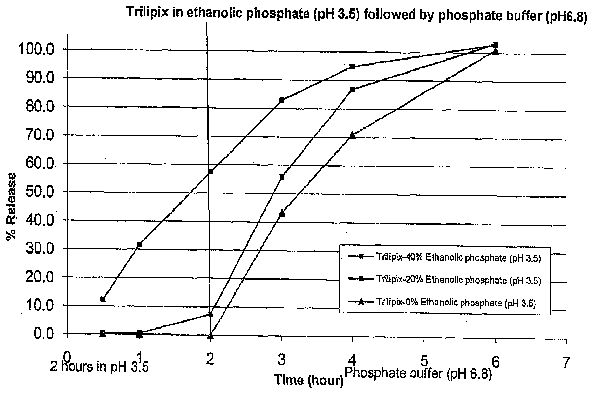

The dissolution characteristics of TriLipix® (choline fenofibrate delayed release capsules for oral administration) were studied. Each delayed release capsule contains enteric coated mini-tablets comprised of choline fenofibrate. Fenofibric acid, active metabolite of choline fenofibrate, has higher aqueous solubility than fenofibrate at alkaline pH. The FDA and Abbott agreed that a representative dissolution/release testing in acid (pH 3.5) is more informative of the drug activity. See NDA 22-224 Clinical Pharmacology and Biopharmaceutics section 2.6, pages 46-48. Accordingly, ccommercially available TriLipix® (delayed release capsules) released about 8% of the drug at 2 hrs in 20% ethanolic acid (pH 3.5) (USP Apparatus II), and released greater than 58% of the drug at 2 hrs in 40% ethanolic acid (pH 3.5)(USP Apparatus II). See Figure 9. Subsequent dissolution in phosphate buffer (pH 6.8) demonstrates that 100% of the drug was released from the delayed release formulation after 6 hours.

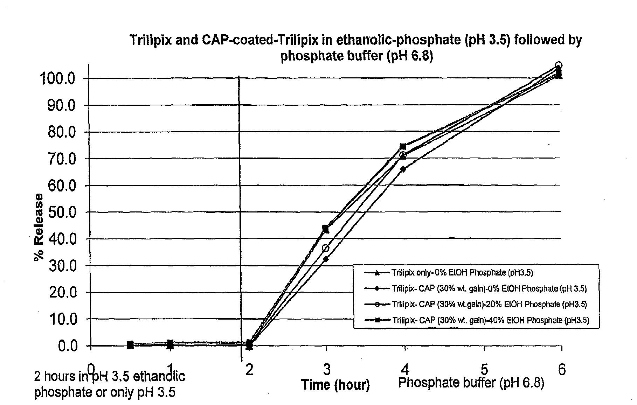

TriLipix® mini-tablets were coated with Cellulose Acetate Phthalate (CAP) solvent-dispersion in an amount of about 30% weight gain. Figure 10 shows the dissolution and release of fenofibric acid for this coated formulation. No measurable drug was released at 2 hrs in 0%, 20%, and 40% ethanolic acid (pH 3.5) (USP Apparatus II). Subsequent dissolution in phosphate buffer (pH 6.8) demonstrates that 100% of the drug was released from the delayed release formulation after 6 hours.

### EXAMPLE 14

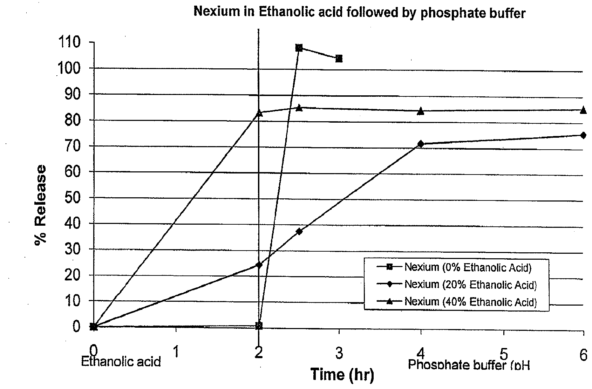

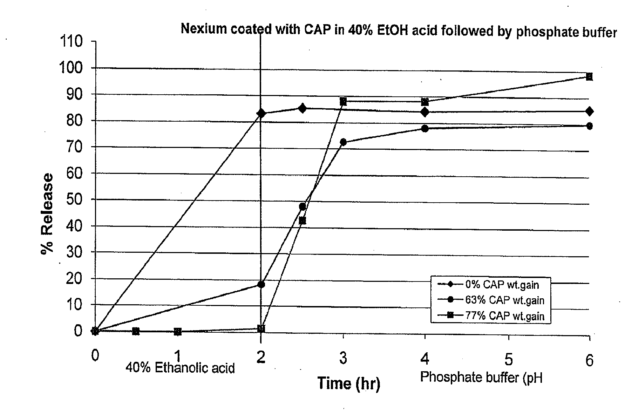

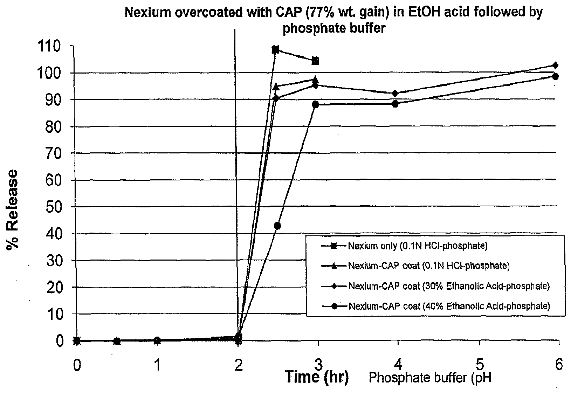

Nexium ® beads were studied in 20% and 40% ethanolic acid (Figure 11) and complete dose dumping was observed in 40% ethanolic acid. Nexium ® beads were coated with a similar cellulose acetate phthalate solvent-dispersion (63% weight gain) as described in Example 6. This formulation released 20% of the drug in 40% ethanolic HCl and 80% of the drug was released in phosphate buffer pH 6.8 (Figure 12). Nexium ® beads were also coated with cellulose acetate phthalate solvent-dispersion to obtain a 77% wt. gain, which released 1.5% of the drug in 40% ethanolic HCl and 90% drug was released in phosphate buffer pH 6.8 (See also Figure 12). Nexium® beads and CAP-coated Nexium® beads (77% weight gain) did not release a measurable amount of drug in 0.1N HCl and 90% of the drug was released in phosphate buffer pH 6.8 (Figure 13). CAP coated Nexium® beads (77% weight gain) did not release a measurable amount of drug in 30% ethanolic HCl and 90% of the drug was released in phosphate buffer pH 6.8 (Figure 13).

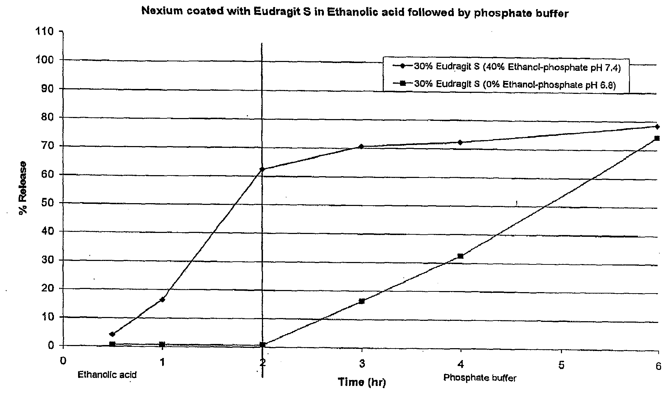

Nexium® beads coated with Eudragit® S solvent-dispersion (to a 30% weight gain), which released 60% drug in 40% ethanolic HCl (Figure 14). To prepare the Eudragit® S dispersion, the materials shown in the Table 11 were mixed in a low shear mixer. Water and IPA was added slowly until the mixture dissolved. Triethyl citrate and talc were added and stirred for 12-15 min.

**Table 11**

| Material | Composition (g) |
|---|---|
| Eudragit® S | 7.5 |
| Triethyl Citrate | 0.8 |
| Talc | 3.7 |
| Purified Water | 3.0 |
| Acetone | 34.0 |

The beads were coated using a fluidised bed coater.

## Claims

1. An alcohol-resistant pharmaceutical composition comprising:
(i) an active agent;
(ii) an enteric system; and
(iii) an alcohol protectant in an amount sufficient to prevent release of the active agent in the presence of alcohol, the alcohol protectant being organic based cellulose acetate phthalate,
wherein the alcohol protectant is present in the pharmaceutical composition in an amount that provides a percentage weight gain ranging from 10% to 500%, and wherein the percentage of active agent released is less than or about 35% in 40% ethanolic HCl in 2 hrs.

2. The composition of claim 1, wherein the release of the active agent in the presence of alcohol is defined by a percentage of active agent released, which percentage is selected from the group consisting of less than or about 1%, 2%, 5%, 8%, 10%, 15%, 20%, 25%, 30% in 40% ethanolic HCl in 2 hours.

3. The composition of claim 1, wherein the release of the active agent in the presence of alcohol is defined by a percentage of active agent released, which percentage is selected from the group consisting of less than or about 1%, 2%, 5%, 8%, 10%, 15%, 20%, 25%, 30% in 20% ethanolic HCl in 2 hours.

4. The composition of claim 1, wherein an amount selected from the group consisting of more than or about 1%, 2%, 5%, 8%, 10%, 15%, 20%, 25%, 30%, 35%, 40%, 45%, 50%, 55%, 60%, 65%, 70%, 75%, 80%, 85%, 90%, 95%, and 99% of the active agent is released from the composition in phosphate buffer (pH 6.8) in 4 hours.

5. The composition of claim 3, wherein the percentage of active agent released is less than or about 25% in 20% ethanolic HCl in 2 hrs.

6. The composition of claim 1, wherein the active agent selected from the group consisting of duloxetine HCl, esomeprazole, rabeprazole sodium, mesalamine, budesonide, lamotrigine, dexlansoprazole, pancreatin, pancrelipase, divalproex sodium, omeprazole, lanzoprazole, diclofenac sodium, valproic acid, fenofibric acid, didanosine, aspirin, bisacodyl, naproxen, erythromycin, sodium rabeprazole, adenovirus vaccine type 4, calcitonin, darapladib, mesalzine, alendronic acid, eprotirome, NE-F (Nephritic factor), glatiramer, CH-1504, bisphosphonate (zoledronic acid) compound, mercaptamine, larazotide, oral insulin, and mixtures or combinations thereof.

7. The composition of claim 1, wherein the enteric system is incorporated into the composition in a form selected from the group consisting of a coating, a layer, a matrix, and combinations thereof.

8. The composition of claim 1, wherein the enteric system comprises components selected from the group consisting of aqueous and organic based hydroxyl propyl methyl cellulose acetate succinate, poly vinyl acetate phthalate, organic based cellulose acetate phthalate, and poly(methacylic acid-co-ethyl acrylate) anionic copolymers.

9. The composition of claim 1, further comprising a disintegrant selected from the group consisting of a swellable material, a superdisintegrant, and mixtures or combinations thereof.

10. The composition of claim 1, further comprising a barrier material disposed between the active agent and the alcohol protectant.

11. The composition of any preceding claim, wherein the enteric system and the alcohol protectant are embodied in a combination of materials or polymers combined in an excipient mixture or embodied in a single polymer system and disposed in a layer, coating or formed into a matrix.

12. The composition of claim 11, wherein the enteric system and the alcohol protectant are provided as a coating of a single polymer system.

13. The composition of any one of claims 1-12, for use in the treatment of a disease, wherein the treatment comprises:
identifying a patient susceptible to concomitant ingestion of alcohol during periods of time which the active agent would reside in the stomach of the patient;
selecting the enteric-coated, alcohol-resistant active agent formulation suitable for treating the disease over a commercially equivalent formulation; and
administering to a patient afflicted with the disease the enteric-coated, alcohol-resistant active agent formulation.

## Patentansprüche

1. Alkoholresistente pharmazeutische Zusammensetzung, die Folgendes umfasst:
(i) einen Wirkstoff,
(ii) ein darmlösliches System und
(iii) einen Alkoholprotektor in einer Menge, die ausreicht, um den Wirkstoff in Gegenwart von Alkohol freizusetzen, wobei der Alkoholprotektor ein Celluloseacetatphthalat auf organischer Basis ist,
wobei der Alkoholprotektor in der pharmazeutischen Zusammensetzung in einer Menge vorliegt, die einen Gewichtsprozentzuwachs im Bereich von 10 bis 500 % bereitstellt und wobei der Anteil des innerhalb von 2 h in 40%iger ethanolischer HCl freigesetzten Wirkstoffs etwa 35 % oder weniger beträgt.

2. Zusammensetzung nach Anspruch 1, wobei die Freisetzung des Wirkstoffs in Gegenwart von Alkohol durch einen Anteil des innerhalb von 2 h in 40%iger ethanolischer HCl freigesetzten Wirkstoffs definiert ist, wobei der Anteil aus der aus weniger als oder etwa 1 %, 2 %, 5 %, 8 %, 10 %, 15 %, 20 %, 25 %, 30 % bestehenden Gruppe ausgewählt ist.

3. Zusammensetzung nach Anspruch 1, wobei die Freisetzung des Wirkstoffs in Gegenwart von Alkohol durch einen Anteil des innerhalb von 2 h in 20%iger ethanolischer HCl freigesetzten Wirkstoffs definiert ist, wobei der Anteil aus der aus weniger als oder etwa 1 %, 2 %, 5 %, 8 %, 10 %, 15 %, 20 %, 25 %, 30 % bestehenden Gruppe ausgewählt ist.

4. Zusammensetzung nach Anspruch 1, wobei eine aus der aus mehr als oder etwa 1 %, 2 %, 5 %, 8 %, 10 %, 15 %, 20 %, 25 %, 30 %, 35 %, 40 %, 45 %, 50 %, 55 %, 60 %, 65 %, 70 %, 75 %, 80 %, 85 %, 90 %, 95 % und 99 % bestehenden Gruppe ausgewählte Menge an Wirkstoff aus der Zusammensetzung in Phosphatpuffer (pH 6,8) innerhalb von 4 h freigesetzt wird.

5. Zusammensetzung nach Anspruch 3, wobei der Anteil an Wirkstoff, der innerhalb von 2 h in 20%iger ethanolischer HCl freigesetzt wird, weniger als oder etwa 25 % beträgt.

6. Zusammensetzung nach Anspruch 1, wobei der Wirkstoff aus der aus folgenden bestehenden Gruppe ausgewählt ist: Duloxetin-HCl, Esomeprazol, Rabeprazolnatrium, Mesalamin, Budesonid, Lamotrigin, Dexlansoprazol, Pancreatin, Pancrelipase, Divalproex-Natrium, Omeprazol, Lanzoprazol, Diclofenac-Natrium, Valproinsäure, Fenofibrinsäure, Didanosin, Aspirin, Bisacodyl, Naproxen, Erythromycin, Natriumrabeprazol, Adenovirus-Vakzine Typ 4, Calcitonin, Darapladib, Mesalzin, Alendronsäure, Eprotirom, NE-F (nephritischer Faktor), Glatiramer, CH-1504, Biphosphonat- (Zoledronsäure-) Verbindung, Mercaptamin, Larazotid, oralem Insulin und Gemischen oder Kombinationen davon.

7. Zusammensetzung nach Anspruch 1, wobei das darmlösliche System in die Zusammensetzung in einer Form miteinbezogen ist, die aus der aus einer Beschichtung, einer Schicht, einer Matrix und Kombinationen davon bestehenden Gruppe ausgewählt ist.

8. Zusammensetzung nach Anspruch 1, wobei das darmlösliche System Komponenten umfasst, die aus der aus Hydroxylpropylmethylcelluloseacetatsuccinat auf wässriger und organischer Basis, Polyvinylacetatphthalat, Celluloseacetatphthalat auf organischer Basis und anionischen Poly(methacrylsäure-co-ethylacrylat)-Copolymeren bestehenden Gruppe ausgewählt sind.

9. Zusammensetzung nach Anspruch 1, die außerdem ein Zersetzungsmittel umfasst, das aus der aus einem quellbaren Material, einem Superzersetzungsmittel und Gemischen und Kombinationen davon bestehenden Gruppe ausgewählt ist.

10. Zusammensetzung nach Anspruch 1, die außerdem ein Barrierematerial umfasst, das zwischen dem Wirkstoff und dem Alkoholprotektor angeordnet ist.

11. Zusammensetzung nach einem der vorangegangenen Ansprüche, wobei das darmlösliche System und der Alkoholprotektor in einer Kombination von Materialien oder Polymeren ausgeführt sind, die in einem Exzipientengemisch kombiniert sind, oder in einem Einzelpolymersystem ausgeführt und in einer Schicht, einer Beschichtung angeordnet oder zu einer Matrix geformt ist.

12. Zusammensetzung nach Anspruch 11, wobei das darmlösliche System und der Alkoholprotektor als Beschichtung eines Einzelpolymersystem bereitgestellt sind.

13. Zusammensetzung nach einem der Ansprüche 1 bis 12 zur Verwendung zur Behandlung einer Erkrankung, wobei die Behandlung Folgendes umfasst:
das Identifizieren eines Patienten, bei dem die Gefahr einer gleichzeitigen Einnahme von Alkohol in Zeiträumen, in denen der Wirkstoff im Magen des Patienten vorliegt, besteht;
das Auswählen der darmlöslich beschichteten, alkoholresistenten Wirkstoffformulierung, die zur Behandlung der Erkrankung geeignet ist, anstelle einer im Handel erhältlichen gleichwertigen Formulierung; und
das Verabreichen der darmlöslich beschichteten, alkoholresistenten Wirkstoffformulierung an einen Patienten, der an der Erkrankung leidet.

## Revendications

1. Composition pharmaceutique résistante à l'alcool comprenant :
(i) un agent actif ;
(ii) un système entérique ;
(iii) un agent protecteur d'alcool en une quantité suffisante pour empêcher la libération de l'agent actif en présence d'alcool, le protecteur d'alcool étant de l'acétophtalate de cellulose à base organique,
dans laquelle l'agent protecteur d'alcool est présent dans la composition pharmaceutique en une quantité qui fournit un pourcentage de gain de poids allant de 10 % à 500 %, et dans laquelle le pourcentage d'agent actif libéré est inférieur ou égal à environ 35 % dans du HCl éthanolique à 40 % en 2 heures.

2. Composition selon la revendication 1, dans laquelle la libération de l'agent actif en présence d'alcool est définie par un pourcentage d'agent actif libéré, lequel pourcentage est choisi dans le groupe constitué de moins que ou d'environ 1 %, 2 %, 5 %, 8 %, 10 %, 15 %, 20 %, 25 %, 30 %, dans du HCl éthanolique à 40 % en 2 heures.

3. Composition selon la revendication 1, dans laquelle la libération de l'agent actif en présence d'alcool est définie par un pourcentage d'agent actif libéré, lequel pourcentage est choisi dans le groupe constitué de moins que ou d'environ 1 %, 2 %, 5 % 8 %, 10 %, 15 %, 20 %, 25 %, 30 %, dans du HCl éthanolique à 20 % en 2 heures.

4. Composition selon la revendication 1, dans laquelle une quantité choisie dans le groupe constitué de plus que ou d'environ 1 %, 2 %, 5 %, 8 %, 10 %, 15 %, 20 %, 25 %, 30 % 35 %, 40 %, 45 %, 50 %, 55 %, 60 %, 65 %, 70 %, 75 %, 80 %, 85 %, 90 %, 95 % et 99 % de l'agent actif est libérée de la composition dans un tampon de phosphate (pH 6,8) en 4 heures.

5. Composition selon la revendication 3, dans laquelle le pourcentage d'agent actif libéré est inférieur ou égal à environ 25 % dans du HCl éthanolique à 20 % en 2 heures.

6. Composition selon la revendication 1, dans laquelle l'agent actif est choisi dans le groupe constitué de la duloxétine HCl, de l'ésoméprazole, du rabéprazole sodique, de la mésalamine, du budésonide, de la lamotrigine, du dexlansoprazole, de la pancréatine, de la pancrélipase, du divalproex sodique, de l'oméprazole, du lanzoprazole, du diclofénac sodique, de l'acide valproïque, de l'acide fénofibrique, de la didanosine, de l'aspirine, du bisacodyl, du naproxène, de l'érythromycine, du rabéprazole sodique, du vaccin adénoviral de type 4, de la calcitonine, du darapladib, de la mésalzine, de l'acide alendronique, de l'éprotirome, du NE-F (facteur néphritique), du glatiramère, du CH-1504, du composé de bisphosphonate (acide zolédronique), de la mercaptamine, du larazotide, de l'insuline orale et de mélanges ou combinaisons de ceux-ci.

7. Composition selon la revendication 1, dans laquelle le système entérique est incorporé dans la composition sous une forme choisie dans le groupe consistant en un revêtement, une couche, une matrice et des combinaisons de ceux-ci.

8. Composition selon la revendication 1, dans laquelle le système entérique comprend des composants choisis dans le groupe constitué par l'acétosuccinate d'hydroxypropylméthylcellulose à base aqueuse et organique, le phtalate d'acétate de polyvinyle, l'acétophtalate de cellulose à base organique et les copolymères anioniques de poly(acide méthacyclique-co-acrylate d'éthyle).

9. Composition selon la revendication 1, comprenant en outre un agent désintégrant choisi dans le groupe constitué d'un matériau pouvant gonfler, d'un super-désintégrant, et de mélanges ou de combinaisons de ceux-ci.

10. Composition selon la revendication 1, comprenant en outre un matériau de barrière disposé entre l'agent actif et l'agent protecteur d'alcool.

11. Composition selon l'une quelconque des revendications précédentes, dans laquelle le système entérique et l'agent protecteur d'alcool sont réalisés sous la forme d'une combinaison de matériaux ou de polymères combinés dans un mélange d'excipients ou réalisés sous la forme d'un système polymère unique et disposés dans une couche, un revêtement ou formés en une matrice.

12. Composition selon la revendication 11, dans laquelle le système entérique et l'agent protecteur d'alcool sont fournis sous la forme d'un revêtement d'un système polymère unique.

13. Composition selon l'une quelconque des revendications 1 à 12, destinée à être utilisée dans le traitement d'une maladie, dans lequel le traitement comprend les étapes consistant à :
identifier un patient susceptible d'une ingestion concomitante d'alcool pendant des périodes où l'agent actif résiderait dans l'estomac du patient ;
sélectionner la formulation d'agent actif résistante à l'alcool et à enrobage entérique appropriée pour traiter la maladie par rapport à une formulation commercialement équivalente ; et
administrer à un patient atteint de la maladie la formulation d'agent actif résistante à l'alcool et à enrobage entérique.
